# EUROPEAN PATENT APPLICATION

(11) **EP 4 101 460 A1**
(43) Date of publication of application: **14.12.2022**
(21) Application number: 22178134.7
(22) Date of filing: 09.06.2022
(51) Int. Cl.: A61K 36/74, A61K 31/07, A61K 31/375, A61K 31/355, A61K 33/04, A61K 33/06, A61K 33/30, A61P 1/00, A61P 19/00, A61P 21/00, A61P 29/00, A61P 37/04, A61K 31/00, A61K 33/00, A61K 31/593

(54) **PHYTOTHERAPY COMPOSITION ASSOCIATED TO POLYVITAMIN AND MINERAL SUPPLEMENTS**

(30) Priority: 09.06.2021 BR 102021011153
(71) Applicant: Campos Barbosa, Fernando, 04119-060 São Paulo (BR); Pereira Rezende, Janaina Drawanz, 04191-190 São Paulo (BR)
(72) Inventor: Campos Barbosa, Fernando, 04119-060 São Paulo (BR); Pereira Rezende, Janaina Drawanz, 04191-190 São Paulo (BR)
(74) Representative: Torner, Juncosa I Associats, SL

(57) **Abstract**

This invention relates to a phytotherapic composition associated to polyvitamin and mineral supplements in anti-inflammatory, antioxidant, immunostimulant treatments, as well as helping treatments of viral infections, musculoskeletal inflammatory disorders, and gastrointestinal disorders with lesions, in addition to microbiota alterations.

## Description

### Field of the invention

This invention relates to a phytotherapic composition associated to polyvitamin and mineral supplements in anti-inflammatory, antioxidant, immunostimulant treatment, as well as helping with treatments of viral infections, musculoskeletal inflammatory disorders, and gastrointestinal disorders with lesions, as well as microbiota alterations.

### Background of the invention

Currently, there are no phytotherapic products associated to polyvitamin and mineral supplements. Thus, the inventors of this invention noted the need to develop a phytotherapic composition associated to polyvitamin and mineral supplements in order to enable synergic action among the substances, aiming particularly at health maintenance and aiding and supplementing treatments. The presence of these bioactive components fosters biological and physiological actions for promoting and maintaining health in the human organism.

This phytotherapic composition associated to polyvitamins and minerals is safe and easy to access to the population, obtained with no need for medical prescriptions. However, it extends the therapeutic arsenal of medical practitioners by supplementing their prescriptions.

The new proposed solution works with the underlying metabolic condition, such as the inflammatory function, to maintain health with quality of life.

The advantages of this composition consist of functional synergism in the action of its bioactive compounds, promoting anti-inflammatory, antioxidant, and immunostimulant actions, in addition to aiding in treatments of viral infections, musculoskeletal inflammatory disorders, and gastrointestinal disorders of injuries, as well as microbiota alterations.

### Brief description of this invention

This invention provides a phytotherapic composition associated to polyvitamin and mineral supplements comprising (i) *Uncaria tomentosa;* (ii) a vitamin complex, comprised of vitamin A, vitamin C, vitamin D, vitamin E; (iv) selenium; (v) zinc; and (vi) magnesium.

Compositions containing *Uncaria tomentosa* (Willd. DC.), also known as cat's claw, are ranked as traditional phytotherapic products. The clinical dose is well established and described in the Phytotherapic Memento (Memento Fitoterápico) (Brazilian Pharmacopoeia, 1st edition) as 350 mg dry extract twice daily. The parts commonly used are the roots and stems, from which powders and extracts are obtained. Its actions are related to positive modulation of the immune system, with antiviral and antibacterial mechanisms described, in addition to anti-inflammatory and antioxidant activities. It includes more than fifty compounds with biological activities, in three main classes: alkaloids, quinovinic acid glycosides, and polyhydroxylated triterpenes. The dosage and form of administration reflect traditional uses as adjunctive therapy enhancing the body's immune capacity to fight a variety of inflammatory conditions.

In addition to taking cat's claw to maintain health and supplementing treatments, a use is proposed for a composition of certain vitamins and minerals with the same purpose, of aiding the maintenance of immune and anti-inflammatory functions.

Appropriate vitamin supplementation is necessary to maintain normal cell function and renewal, and to promote tissue repair. There is growing evidence that specific vitamin requirements increase during times of stress, due to greater loss or high use. Additionally, several vitamins play important roles in regulating immune responses and promoting tissue healing.

Vitamin and mineral deficiencies are subclinical, i.e., with no apparent symptomatology, until chronic deficiency situations can be clinically proven.

The antioxidant network inactivates or eliminates free radicals, thus maintaining the reduction-oxidation balance and preventing oxidation damage. However, if antioxidant defenses are limited or radical production becomes excessive and outstrips defense capacity, the balance is disturbed, and oxidative stress occurs.

Radicals react with molecules such as lipids, proteins, and DNA. This can result in lipid peroxidation, enzyme inactivation, and DNA base modification, with strand breaks and cross-linking. If the damage is severe, it can result in cell death, cell damage, tissue damage, and organ dysfunction. Oxidative stress-lead to d damage is associated with acute and chronic conditions as a causative agent or as a consequence of free radical generation. The use of the components listed below is thus proposed for preventing deficiencies in processes involving the immune system, which are common when large consumption or loss by the body occurs.

Vitamins A, C, D, and E have effects on the immune function. Vitamins A and C have an effect on healing, and vitamins A, C, and E have a defense function against free radical damage.

Vitamin A or retinol acts as an antioxidant in the lipid peroxidation process. Retinol also potentiates the antioxidant effects of ascorbic acid.

Vitamin C, or ascorbic acid, is an antioxidant and a cofactor for several enzymes. It is involved in iron and folic acid metabolism, as well as collagen, cortisol, catecholamines, and carnitine synthesis. Vitamin C is also found in high concentrations in leukocytes and can boost the immune system through several pathways.

Vitamin C acts indirectly as an antioxidant, serving as a substrate for ascorbate peroxidase, protecting the endothelium against phagocyte adhesion and thus preventing oxidation and damage to the endothelium caused by free radicals generated by phagocytes.

In addition to antioxidant activity, Vitamin E maintains cell membrane stability and thus immune responses to infection. α-Tocopherol is considered the most important lipid-soluble antioxidant in cell membranes, protecting them from lipid peroxidation.

In its active form, selenium is associated with cysteine and methionine (selenocysteine and selenomethionine) and is an essential component of selenoproteins (e.g., selenoprotein P, glutathione peroxidase - GSHPx etc.). Selenium is an essential micronutrient and functions as an enzymatic cofactor of more than thirty selenoproteins, which have numerous biological functions, related especially to redox signaling, the antioxidant defense system, the thyroid hormone metabolism, and humoral and cell-mediated immune responses.

Zinc has mediated action and importance as a cofactor of approximately 120 enzymes (carbonic anhydrase, carboxypeptidase, alkaline phosphatase, oxidoreductases, transferases, ligases, hydrolases, lyases, and isomerases), in addition to being an integral constituent of DNA polymerase, reverse transcriptase, RNA polymerase, tRNA synthetase, and the protein chain elongation factor.

Zinc deficiency has a pronounced effect on nucleic acid metabolism and influences protein and amino acid metabolism. The mechanism involves changes in the nature of RNA polymerase and mRNA composition with consequences on histone synthesis.

Zinc is an essential trace element required for proper immune system function, glucose control, neurocognitive function, wound healing, and oxidative stress response. It has no direct mechanism that acts on free radicals, but exerts effects on metabolic mechanisms involved in their formation.

Zinc deficiency impairs immune system responses, as it results in decreased T and B cell maturation; which leads to lymphopenia; reduces natural killer cell proliferation and phagocytic cell function; and alters cytokine responses. It also modifies the barrier functions of the skin, lungs, and gastrointestinal tract. In the lungs, it also alters lipid metabolism, which can result in pulmonary edema. Zinc plays an important role in antioxidant defense, and its deficiency can thus disrupt the oxidant-antioxidant balance and lead to oxidative stress. As oxidative stress plays an important role in the pathophysiology of organ failure, it may indirectly contribute to multiple organ dysfunction.

Magnesium is the fourth most abundant cation in the human body. Of all the magnesium found in the human body, about 50% - 60% is located in bones and approximately 1% is located in extracellular fluid. The remaining magnesium is contained in the cells, making it the second most abundant intracellular cation (after potassium). Magnesium homeostasis is regulated by a number of factors, including vitamin D-controlled absorption from the gastrointestinal tract (GIT) and renal excretion. Normally, 30% - 40% of dietary magnesium is absorbed. Part of magnesium homeostasis is dependent on renal excretion, which is influenced by sodium and calcium excretion, extracellular fluid volume, and the PTH hormone, whose release causes the excretion of magnesium, calcium or sodium and decreased plasma volume.

In the immunological context, early observations revealed a direct correlation between Mg2+ deficiency and an increase in systemic inflammation based on elevated serum levels of tumor necrosis factor (TNF)-a and other pro-inflammatory cytokines and reduced concentrations of anti-inflammatory cytokines. There is also potential involvement in membrane receptors involved in immune system cell signaling.

Mg2+ deficiency may lead to different physiological stress reactions that are relevant to the immune response, causing endothelial dysfunction and inflammatory syndromes accompanied by leukocyte and macrophage activations, as well as increased pro-inflammatory cytokines, acute phase proteins and free radicals. On the other hand, magnesium sulfate supplementation may mediate anti-inflammatory effects through activation of phosphoinositide 3-kinase (PI3K, an enzyme family involved in cellular functions such as cell growth, proliferation, differentiation, motility, survival, and intracellular traffic) and inhibition of L-type ion channels. In addition, Mg2+ deficiency affects the proliferation of mast cells and their histamine storage and secretion function.

### Detailed description of this invention

This invention describes a phytotherapic composition associated to polyvitamin and mineral supplements comprising (i) *Uncaria tomentosa;* (ii) choline; (iii) a vitamin complex, comprising vitamin A, vitamin B6, vitamin C, vitamin D, vitamin E, vitamin B1 and vitamin B12; (iv) selenium; (v) zinc; and (vi) magnesium, wherein: (i) *Uncaria tomentosa* is present in the range of 16% - 20% p.p.; (ii) choline is present in the range of 19% - 23% p.p; (iii) vitamin A is present in the range of 0.25% - 0.65% p.p., vitamin B6 is present in the range of 0.19% - 0.23% p.p., vitamin C is present in the range of 41% -45% p.p., vitamin D is present in the range of 0.001% - 0.003% p.p., vitamin E is present in the range of 11% - 15% p.p., vitamin B1 is present in the range of 0.11% - 0.15% p.p., and vitamin B12 is present in the range of 0.0002% - 0.0004% p.p.; (iv) selenium is present in the range of 0.011% - 0.015% p.p.; (v) zinc is present in the range of 1% - 3% p.p.; and (vi) magnesium is present in the range of 0.009% - 0.013% p.p., with the range based on 100% of the total weight of the composition.

Preferably this invention describes a phytotherapic composition associated to polyvitamin and mineral supplements comprising (i) 18.5% p.p. *Uncaria tomentosa;* (ii) 21.7% p.p. choline; 0.45% p.p. is vitamin A, 0.21% p.p. is vitamin B6, 43.7% p.p. p.p. is vitamin C, 0.002% p.p. is vitamin D, 13% p.p. is vitamin E, 0.13% p.p. is vitamin B1; 0.0003% p.p. is vitamin B12; (iv) 0.013% p.p. of selenium; (v) 2.4% p.p. of zinc; and (vi) 0.01% p.p. of magnesium, with the range based on 100% of the total weight of the composition.

The phytotherapic composition associated to polyvitamin and mineral supplements addressed by this invention is prepared in powder form, suitable for encapsulation and oral administration.

This invention also provides for the use of the phytotherapic composition associated to polyvitamin and mineral supplements as defined above, in the preparation of an anti-inflammatory, antioxidant, and immunostimulant supplement to help with the treatment of viral infections, musculoskeletal inflammatory disorders and gastrointestinal disorders with lesions, as well as microbiota alterations.

## Claims

1. PHYTOTHERAPIC COMPOSITION ASSOCIATED TO POLYVITAMIN AND MINERAL SUPPLEMENTS, **characterized in that** it comprises: (i) *Uncaria tomentosa*; (ii) a vitamin complex, comprising vitamin A, vitamin C, vitamin D, vitamin E, (iii) selenium; (iv) zinc; and (v) magnesium, where: (i) *Uncaria tomentosa* is present in the range of 42% - 46% p. p; (ii) vitamin A is present in the range of 0.03% - 0.07% p.p., vitamin C is present in the range of 30% - 34% p.p., vitamin D is present in the range of 0.0028% - 0.0035% p.p, vitamin E is present in the range of 17% - 21% p.p.; (iv) selenium is present in the range of 0.017% - 0.021% p.p.; (v) zinc is present in the range of 1% - 5% p.p.; and (vi) magnesium is present in the range of 0.014% - 0.018% p.p., with the range based on 100% of the total weight of the composition.

2. PHYTOTHERAPIC COMPOSITION, according to claim 1, **characterized in that** it comprises: (i) 44.9 % p.p. *Uncaria tomentosa;* (ii) a vitamin complex wherein 0.06% p.p. is vitamin A, 32% p.p. is vitamin C, 0.003% p.p. is vitamin D, 19% p.p. is vitamin E; (iii) 0.02% p.p. is selenium; (iv) 3.8% p.p. is zinc; and (v) 0.017% p.p. is magnesium, with the range based on 100% of the total weight of the composition.

3. PHYTOTHERAPIC COMPOSITION, according to claims 1 and 2, **characterized in that** it is prepared in powder form, suitable for encapsulation and oral administration.

4. USE OF A PHYTOTHERAPIC COMPOSITION ASSOCIATED TO POLYVITAMIN AND MINERAL SUPPLEMENTS as defined by one of claims 1 to 3, **characterized in that** it is used in the preparation of an anti-inflammatory, antioxidant, immunostimulant supplement to help treatments of viral infections, musculoskeletal inflammatory disorders, and gastrointestinal disorders with lesions, as well as microbiota alterations.
